# EUROPÄISCHE PATENTSCHRIFT

(11) **EP 2 146 638 B1**
(45) Veröffentlichungstag und Bekanntmachung des Hinweises auf die Patenterteilung: **24.10.2012**
(21) Anmeldenummer: 08748902.7
(22) Anmeldetag: 14.04.2008
(51) Int. Cl.: A61B 5/151

(54) **STECHSYSTEM MIT LANZETTENTRÄGERBAND**
PRICKING SYSTEM WITH A LANCET CARRIER STRIP
SYSTÈME DE PIQÛRE AVEC UNE BANDE DE SUPPORT POUR LANCETTES

(30) Priorität: 10.05.2007 EP 07009367
(43) Veröffentlichungstag der Anmeldung: 27.01.2010
(73) Patentinhaber: Roche Diagnostics GmbH, 68305 Mannheim (DE); F. Hoffmann-La Roche AG, 4070 Basel (CH)
(72) Erfinder: KONYA, Ahmet, 67165 Waldsee (DE); DECK, Frank, 67150 Niederkirchen (DE); LIST, Hans, 64754 Hesseneck-Keilbach (DE); KEIL, Michael, 67071 Ludwigshafen (DE); HÖRAUF, Christian, 68723 Oftersheim (DE); WEISS, Thomas, 68307 Mannheim (DE); SCHLOTHAUER, Marcus, 99817 Eisenach (DE); KUHR, Hans-Jürgen, 68219 Mannheim (DE)
(74) Vertreter: Mommer, Niels
(86) Internationale Anmeldenummer: PCT/EP2008/002923
(87) Internationale Veröffentlichungsnummer: WO 2008/138443

(56) Entgegenhaltungen:
- EP-A- 1 714 613
- EP-A- 1 873 521
- EP-A1- 1 360 935
- WO-A-2004/060174
- WO-A1-2005/104948
- DE-B1- 2 803 345
- US-A- 4 725 016
- US-A1- 2005 245 845
- US-A1- 2006 079 811

## Beschreibung

Die Erfindung geht aus von einem Stechsystem mit den im Oberbegriff des Anspruchs 1 angegebenen Merkmalen, wie es aus der DE 2803345 bekannt ist.

Zu einem derartigen Stechsystem gehört als Lanzettenvorrat ein Trägerband, das mehrere Lanzetten trägt. Diese Lanzetten werden durch Bewegen des Trägerbandes in einer Förderrichtung nacheinander in eine Gebrauchsposition gebracht, in der sie mit einem Stechantrieb für eine Stechbewegung beschleunigt werden können, um in einem an eine Gehäuseöffnung des Stechsystems angelegten Körperteil eine Stichwunde zur Gewinnung einer Körperflüssigkeitsprobe zu erzeugen.

Derartige Stechsysteme werden beispielsweise von Diabetikern verwendet, die mehrmals täglich ihren Blutzuckerspiegel überprüfen müssen und dafür eine Körperflüssigkeitsprobe, in der Regel Blut oder interstitielle Flüssigkeit, benötigen, die aus einer mit einem Stechsystem erzeugten Stichwunde gewonnen wird.

Im Gegensatz zu Stechsystem mit Trommelmagazinen, die typischerweise nur sechs oder acht Lanzetten enthalten, kann ein Trägerband einen Lanzettenvorrat mit einer wesentlich größeren Anzahl von Lanzetten bilden. Stechsysteme mit einem Lanzettenvorrat in Form eines Trägerbands haben deshalb den Vorteil, dass sich ein Benutzer seltener um den Austausch eines Trägerbandes oder, bei Verwendung von Wegwerfgeräten, um ein neues Gerät bemühen muss.

Damit mit einer Lanzette eines Trägerbandes in ein an eine Gehäuseöffnung angelegtes Körperteil eingestochen werden kann, muss die Lanzette in einer Gebrauchsposition in Bezug auf die Gehäuseöffnung positioniert werden.

Bei dem aus der DE 2803345 bekannten Stechsystem wird der Transport des Trägerbandes durch Eingreifen von Mitnehmerstiften oder Zähnen eines Zahnrades in äquidistante Durchbrüche des Trägerbandes erreicht. Da die Lanzetten auf dem Trägerband in vorgegeben Positionen relativ zu den Durchbrüchen angeordnet sind, wird durch den mittels der Durchbrüche bewirkten Bandtransport automatisch erreicht, dass die Lanzetten mit ausreichender Präzision in die Gebrauchsposition gelangen.

Ein Stechsystem der eingangs genannten Art ist ferner aus der US 2005/0245845 A1 bekannt. Bei diesem Stechsystem erfolgt der Bandtransport durch eine Wickeleinrichtung, die von Hand oder mittels eines Elektromotors soweit gedreht wird, bis eine neue Lanzette die Gebrauchsposition erreicht hat. Ein automatisiertes System mit einem Elektromotor, der mittels geeigneter Sensoren abgeschaltet wird, sobald eine frische Lanzette die Gebrauchsposition erreicht hat, ist jedoch sehr aufwendig und teuer. Ein manueller Bandtransport, bei dem ein Benutzer die Bewegung des Trägerbandes überwachen und ein Weiterdrehen der Wickeleinrichtung beenden muss, sobald eine frische Lanzette die Gebrauchsposition erreicht hat, ist jedoch wenig benutzerfreundlich, da es leicht geschehen kann, dass eine Lanzette über die Gebrauchsposition hinausbewegt wird.

Aufgabe der Erfindung ist es, einen Weg aufzuzeigen, wie bei einem Stechsystem der eingangs genannten Art Lanzetten mit geringerem Aufwand präzise in einer Gebrauchsposition positioniert werden können.

Diese Aufgabe wird durch ein Stechsystem mit den im Anspruch 1 angegebenen Merkmalen gelöst. Vorteilhafte Weiterbildungen der Erfindung sind Gegenstand der Unteransprüche.

Ein erfindungsgemäßes Stechsystem hat eine lösbare Sperre, die einen Weitertransport des Trägerbandes blockiert, sobald eine Lanzette die Gebrauchsposition erreicht hat. Das Stechsystem kann ein Stechgerät und austauschbare Trägerbänder mit Lanzetten umfassen. Das Stechsystem kann jedoch auch als ein Stechgerät ausgebildet sein, bei dem ein Austausch eines Trägerbandes nicht vorgesehen ist und das bestimmungsgemäß entsorgt wird, sobald alle Lanzette des Trägerbands benutzt wurden.

Ein Trägerband , das im erfindungsgemäβen Stechsystem verwendet werden kann, hat Blockadeelemente, die zum Positionieren einer Lanzette in einer Gebrauchsposition mit einer lösbaren Sperre eines Stechgeräts zusammenwirken. Diese Blockadeelemente können beispielsweise Löcher sein, in welche die Sperre eingreift, um einen Weitertransport des Trägerbandes zu verhindern. Die Blockadeelemente können beispielsweise auch als quer zur Längsrichtung des Trägerbandes verlaufende Stufen ausgebildet sein, die an einen Anschlag des Stechgeräts anschlagen, sobald eine Lanzette eine Gebrauchsposition erreicht hat.

Bei einem erfindungsgemäßen Stechsystem wird eine Fördereinrichtung, mit der die Lanzetten durch Bewegen des Trägerbandes nacheinander in eine Gebrauchsposition gebracht werden können, bevorzugt über eine Rutschkupplung angetrieben. Sobald eine Lanzette ihre Gebrauchsposition erreicht hat, wird ein Weitertransport des Bandes durch eine Sperre blockiert. Die Rutschkupplung rutscht daraufhin durch.

Dies hat den Vorteil, dass eine Antriebsbewegung, die mit einer Betätigungseinrichtung zum Antreiben der Fördereinrichtung erzeugt wird, nicht auf den zum Positionieren einer Lanzette in der Gebrauchsposition erforderlichen Förderweg abgestimmt sein muss und deshalb auch größer sein kann. Der Antrieb der Fördereinrichtung eines erfindungsgemäßen Stechsystems kann deshalb vorteilhaft einfach aufgebaut sein und beispielsweise auch für andere Funktionen wie das Spannen einer Feder eines Stechantriebs, mit dem eine in der Gebrauchsposition positionierte Lanzette für eine Stechbewegung zu beschleunigt wird, genutzt werden.

Rutschkupplungen werden auch als Sicherheits- oder Überlastkupplungen bezeichnet. Im Rahmen der Anmeldung sind diese Begriffe gleichbedeutend. Derartige Kupplungen öffnen sich, sobald ein angreifendes Drehmoment einen vorgegebenen Grenzwert überschreitet und übertragen folglich nur Drehmomente unterhalb dieses Grenzwerts.

Um eine weitere Lanzette in die Gebrauchsposition zu befördern, kann die Sperre gelöst werden, was beispielsweise durch erneutes Betätigen der Betätigungseinrichtung erreicht werden kann.

Bevorzugt umfasst die Fördereinrichtung eines erfindungsgemäßen Stechsystems eine Wickeleinrichtung, die das Band aufwickelt und auf diese Weise in Förderrichtung bewegt. Bei einer derartigen Wickeleinrichtung ändert sich der zum Transport einer Lanzette in die Gebrauchsposition erforderliche Drehwinkel, da mit zunehmendem Aufwickeln des Trägerbandes wegen des zunehmenden Durchmessers der Bandwicklung der durch eine Umdrehung bewirkte Bandtransport zunimmt. Um die zum Positionieren einer Lanzette erforderliche Drehbewegung der Wickeleinrichtung zu bewirken, genügt deshalb eine umso kleinere Antriebsbewegung der Betätigungseinrichtung je mehr Lanzetten eines Trägerbandes bereits benutzt wurden. Mit einer Rutschkupplung kann hierfür in vorteilhafter Weise eine immer wieder gleiche Antriebsbewegung der Betätigungseinrichtung genutzt werden, da nach Blockieren des Weitertransports die Rutschkupplung durchrutscht und die Fördereinrichtung von der restlichen Antriebsbewegung der Betätigungseinrichtung nicht mehr beeinflusst wird.

Eine gleich bleibende Antriebsbewegung lässt sich beispielsweise dadurch bewirken, dass die Betätigungseinrichtung eine Stange umfasst, die zur Betätigung von einem Benutzer in ihrer Längsrichtung verschoben wird. Im einfachsten Fall entspricht die Länge der Antriebsbewegung dabei dem Weg, um den die Stange in Längsrichtung verschoben wird, beispielsweise indem ein aus dem Gehäuse herausragendes Ende der Stange stets um denselben Weg in das Gehäuse gedrückt wird.

Eine Antriebsbewegung der Fördereinrichtung kann beispielsweise auch durch eine Feder bewirkt werden, insbesondere eine Torsionsfeder. Eine Feder, deren Federkraft darauf gerichtet ist, eine Wickelrolle zum Bandtransport in eine gewünschte Drehrichtung zu treiben, kann beispielsweise bei Einlegen einer Bandkassette in das Stechgerät vorgespannt werden oder bei einem Einweggerät, das bestimmungsgemäß nach dem Gebrauch aller Lanzetten seines Trägerbandes entsorgt wird, vom Hersteller vorgespannt eingebaut werden. Ein Stechsystem mit einer derartigen Antriebsfeder lässt sich vorteilhaft kompakt ausbilden, da auf eine Rutschkupplung oder andere Maßnahmen zum Ausgleich eines vom Wickelzustand abhängigen Transportwegs, der durch eine Drehung einer Wickelrolle bewirkt wird, verzichtet werden kann. Die lösbare Sperre eines erfindungsgemäßen Stechsystems lässt in einem solchen Fall nämlich stets nur eine Wickelbewegung zu, bis eine frische Lanzette in die Gebrauchsposition gelangt ist. Ein kurzeitiges Lösen der Sperre kann beispielsweise durch die Mechanik des Stechantriebs bewirkt werden. Insbesondere kann die Rückführbewegung bei einem Lanzettenstich genutzt werden, ein Lösen der Sperre zu bewirken. Bevorzugt wird dabei eine Restenergie des Stechantriebs ausgenutzt, um ein Schaltteil, beispielsweise eine Wippe oder etwas ähnliches, zu betätigen, das die Sperre löst.

Bei einem verhältnismäßig langen Trägerband kann die Verwendung einer vorgespannten Antriebsfeder, die sich schrittweise mit jedem Transportschritt, der eine frische Lanzette in die Gebrauchsposition bringt, entspannt, jedoch zu Problemen führen, da die Federkraft zunächst sehr hoch ist und erst nach Gebrauch einiger Lanzetten abnimmt. Die Festigkeit des Trägerbandes und die Qualität der Antriebsfeder sind in einem solchen Fall hohen Anforderungen ausgesetzt. Um dies zu vermeiden ist deshalb die Antriebsfeder der Fördereinrichtung eines erfindungsgemä-βen Stechsystems bevorzugt mit einem Freilaufmodul, beispielsweise einem Ratschenmechanismus, gekoppelt. Auf diese Weise kann die Antriebsfeder mit einem Betätigungselement vom Benutzer, beispielsweise mittels einer Zahnstange, gespannt werden. Mit einem Freilaufmodul lässt sich nämlich verhindern, dass sich nach einer Spannbewegung des Betätigungselements eine Rückstellbewegung des Betätigungselements auf die Antriebsfeder oder die Wickeleinrichtung überträgt.

Erfindungsgemäβ ist vorgeschen, dass die Sperre als Sperrelement einen Anschlag aufweist, an den eine Lanzette anschlägt, sobald sie die Gebrauchsposition erreicht hat. Auf diese Weise lässt sich eine besonders präzise Positionierung der Lanzetten erreichen. Insbesondere können sogar unregelmäßige Abstände zwischen den Lanzetten eines Trägerbandes ausgeglichen werden, da das Trägerband von der Fördereinrichtung jeweils genau soweit bewegt wird, bis durch den Anschlag der Sperre ein weiterer Bandtransport blockiert ist und folglich eine Lanzette die Gebrauchsposition erreicht hat. Indem bei der Herstellung des Lanzettenträgerbandes größere Toleranzen hinsichtlich der Abstände zwischen den Lanzetten in Kauf genommen werden können, lässt sich zudem die Fertigung vereinfachen und kostengünstiger gestalten.

Bevorzugt wird das Trägerband durch einen Spalt geführt, dessen lichte Weite durch den Anschlag der Sperre begrenzt wird. Für einen Weitertransport des Trägerbandes kann die Sperre gelöst werden, indem die lichte Weite des Spaltes vorübergehend vergrößert wird, so dass ein Bandabschnitt mit einer benutze Lanzette den Spalt passieren kann. Anschließend wird die lichte Weite des Spaltes wieder reduziert, so dass eine frische Lanzette bei Erreichen der Gebrauchsposition an dem Anschlag anschlägt und ein Weitertransport des Bandes blockiert wird. Ein vorübergehendes Öffnen des Spaltes kann beispielsweise durch eine Kulissensteuerung erreicht werden.

Bevorzugt ist der Spalt, dessen lichte Weite durch den Anschlag der Sperre begrenzt wird, in einem Kopplungskopf ausgebildet, der bei einem Lanzettenstich in Stichrichtung bewegt wird. Bevorzugt ist dabei, dass die Lanzetten quer zur Längsrichtung des Trägerbandes angeordnet sind.

In einer bevorzugten Ausführungsform wird eine Antriebsfeder der Fördereinrichtung mit dem gleichen Bedienungsschritt gespannt, der auch die Energie in eine Feder des Stechantriebs einträgt. Hierfür ist ein gemeinsames Bedienungselement besonders vorteilhaft. Das erfolgreiche Spannen beider Federn wird bevorzugt an den Bediener zurückgemeldet durch ein akustisches oder visuelles Signal.

Weitere Einzelheiten und Vorteile der Erfindung werden anhand von Ausführungsbeispielen unter Bezugnahme auf die beigefügten Zeichnungen erläutert. Gleiche und einander entsprechende Bauteile sind dabei mit übereinstimmenden Bezugszahlen gekennzeichnet. Die beschriebenen Merkmale können einzeln oder in Kombination zum Gegenstand von Ansprüchen gemacht werden. Es zeigen:
- Figur 1: eine schematische Darstellung eines Stechsystems;
- Figur 2: ein Ausführungsbeispiel einer Rutschkupplung;
- Figur 3: eine Detailansicht des Stechantriebs mit einem Lanzettenträgerband vor Erreichen der Gebrauchsposition;
- Figur 4: eine Schnittansicht zu Figur 3 entlang der Schnittlinie A-A;
- Figur 5: eine Ansicht gemäß Figur 3 mit einer Lanzette in der Gebrauchsposition;
- Figur 6: eine Schnittansicht zu Figur 5 entlang der Schnittlinie A-A;
- Figur 7: eine Detailansicht des Stechantriebs und eines Trägerbandes vor Erreichen der Gebrauchsposition mit einem alternativen Ausführungsbeispiel eines Sperrmechanismus;
- Figur 8: eine Schnittansicht zu Figur 7 entlang der Schnittlinie A-A;
- Figur 9: eine Ansicht gemäß Figur 7 mit der Lanzette in der Gebrauchsposition;
- Figur 10: eine Schnittansicht zu Figur 9 entlang der Schnittlinie A-A;
- Figur 11: eine schematische Darstellung eines Stechsystems mit einem Aktuator zum Lösen der Sperre;
- Figur 12: ein Ausführungsbeispiel einer Kulisse zur Steuerung des Aktuators;
- Figur 13: eine schematische Darstellung des Aktuators in einer Schnittdarstellung durch das Trägerband;
- Figur 14: eine Darstellung gemäß Figur 13 bei geöffneter Sperre;
- Figur 15: ein weiteres Ausführungsbeispiel eines Stechsystems; und
- Figur 16: ein Ausführungsbeispiel eines Freilaufmoduls.

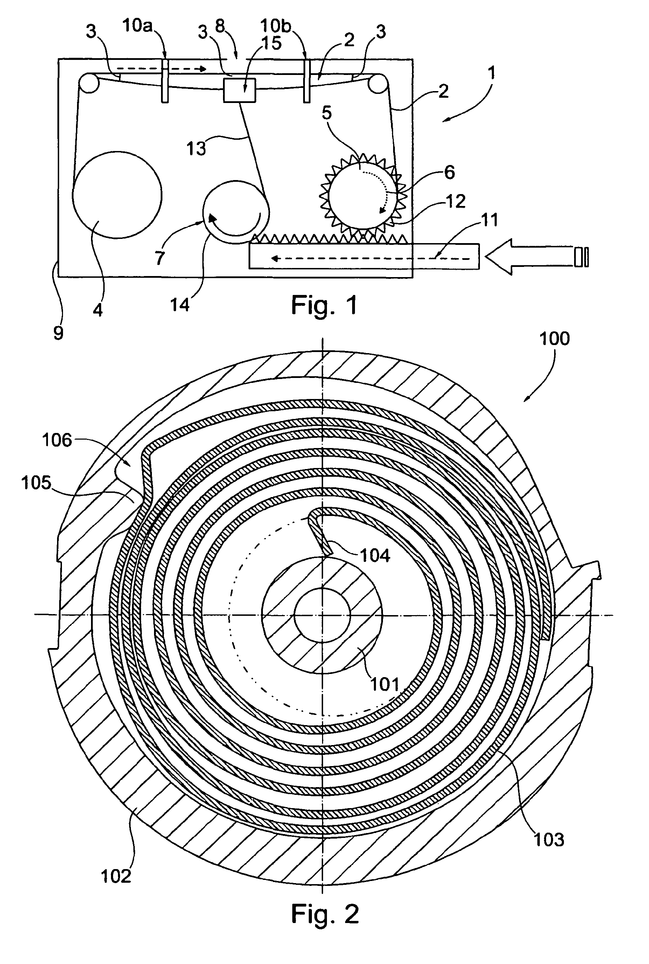

Figur 1 zeigt eine schematische Darstellung eines Stechsystems 1. Zu dem Stechsystem 1 gehört ein Trägerband 2, das mehrere Lanzetten 3 trägt, die quer zur Längsrichtung des Bandes angeordnet sind. Mit einer Fördereinrichtung 6, die bei dem dargestellten Ausführungsbeispiel eine Wickeleinrichtung zum Antreiben einer Rolle 5 ist, kann das Trägerband 2 aufgewickelt und dabei in einer Förderrichtung bewegt werden, so dass die Lanzetten 3 des Trägerbandes 2 nacheinander in eine Gebrauchsposition gelangen, in der sie mit einem Stechantrieb 7 für eine Stechbewegung beschleunigt werden können, um in ein an eine Gehäuseöffnung 8 angelegtes Körperteil einzustechen.

Das Trägerband 2 ist bei dem dargstellten Ausführungsbeispiel ähnlich wie bei einer Tonbandkassette auf eine Vorratsrolle 4 aufgewickelt, von der es abgewickelt und bei Verbrauch der Lanzetten 3 nach und nach auf die angetriebene Wickelrolle 5 aufgewickelt wird. Das Trägerband 2 kann austauschbar in einem Gehäuse 9 eines Stechgeräts angeordnet sein, beispielsweise als Teil einer Kassette, oder in einem Gerät fest eingebaut sein, das bestimmungsgemäß entsorgt wird, sobald alle Lanzetten 3 des Trägerbandes 2 aufgebraucht sind. Anstelle der Vorratsrolle 4 kann beispielsweise auch ein Stapel verwendet werden, zu dem das Trägerband 2 zickzackförmig gefaltet ist.

Beim Bandtransport läuft das Trägerband 2 mit einer Lanzette 3, bevor diese die Gebrauchsposition erreicht, an einer Umlenkeinrichtung 10a vorbei, die ein Verdrehen des Trägerbands 2 um 90° bewirkt. Nach Gebrauch einer Lanzette 3 wird der betreffende Abschnitt des Trägerbandes 2 an einer zweiten Umlenkeinrichtung 10b vorbeigeführt, mit der das Trägerband 2 wieder in seine ursprüngliche Orientierung zurückgedreht wird.

Die Fördereinrichtung 6 wird von einer Betätigungseinrichtung 11 angetrieben, die bei dem dargestellten Ausführungsbeispiel als Zahnstange ausgebildet ist, die mit einem Zahnrad 12 in Eingriff steht. Um eine frische Lanzette 3 in die Gebrauchsposition zu befördern, betätigt ein Benutzer die Betätigungseinrichtung 11, bei dem dargestellten Ausführungsbeispiel indem diese in Richtung des dargestellten Pfeils in das Gehäuse 9 hineingedrückt, also in ihrer Längsrichtung verschoben wird.

Die Betätigungseinrichtung 11 ist über eine Rutschkupplung an die Fördereinrichtung 6 gekoppelt. Bei dem dargestellten Ausführungsbeispiel wirkt die in Figur 2 gezeigte Rutschkupplung zwischen dem Zahnrad 12 und der Wickeleinrichtung 6. Sobald eine Lanzette 3 die Gebrauchsposition erreicht hat, blockiert eine lösbare Sperre einen Weitertransport des Trägerbandes 2. Die Rutschkupplung rutscht daraufhin durch, so dass kein Drehmoment mehr auf die Wickeleinrichtung 6 übertragen wird.

Die Betätigungseinrichtung 11 kann auf diese Weise bei jeder Betätigung eine gleich bleibende Antriebsbewegung durchführen, obwohl sich der zum Transport einer Lanzette 3 in die Gebrauchsposition erforderliche Drehwinkel der Wickeleinrichtung 6 mit zunehmenden Aufwickeln des Trägerbandes 2 auf die Wickelrolle 5 ändert, nämlich abnimmt.

Die Betätigungseinrichtung 11 ist auch mit dem Stechantrieb 7 gekoppelt. Der Stechantrieb 7 hat einen Energiespeicher, der die für eine Stechbewegung erforderliche Energie liefert und durch Betätigen der Betätigungseinrichtung 11 aufgeladen wird. Bei dem dargestellten Ausführungsbeispiel ist der Energiespeicher eine Feder, bevorzugt eine Kunststofffeder, beispielsweise ein elastisches Band, etwa ein Gummiband. Bei dem dargestellten Ausführungsbeispiel erfolgt die Kopplung der Betätigungseinrichtung 11 mit dem Energiespeicher über ein Rad 14, das durch eine Bewegung der Betätigungseinrichtung 11 in Rotation versetzt wird und diese Bewegung über eine Pleuelstange 13 auf einen Kopplungskopf 15 überträgt, der das Trägerband 2 hält.

Figur 2 zeigt ein Ausführungsbeispiel einer Rutschkupplung 100. Die Rutschkupplung 100 hat eine innere Welle 101, die an das in Figur 1 gezeigten Zahnrad 12 gekoppelt ist und eine äußere Welle 102, welche die inner Welle 101 ringförmig umschließt und mit der Wickeleinrichtung 6 des Stechsystems gekoppelt ist. Zwischen der inneren Welle 101 und der äußeren Welle 102 ist eine Spiralfeder 103 angeordnet. Wird die innere Welle 101 gedreht, wird das innere Ende 104 der Spiralfeder 103 mitgenommen und die Spiralfeder 103 verdreht. Das innere Ende 104 kann an der inneren Welle 101 befestigt sein oder lose anliegen, so dass sich eine Bewegung der inneren Welle 101 durch Reibungskräfte auf die Spiralfeder 103 überträgt.

Die Spiralfeder 103 liegt lose an der äußeren Welle 102 an, so dass durch Reibungskräfte ein Drehmoment von der Spiralfeder auf die äußere Welle übertragen werden kann. Diese Reibungskräfte bewirken eine Begrenzung des maximal übertragbaren Drehmoments. Wenn die äußere Welle blockiert, rutscht nämlich die Spiralfeder 103 an der äußeren Welle 102 entlang.

Zur Erhöhung des maximal übertragbaren Drehmoments kann die äußere Welle 102, wie in Figur 2 gezeigt, eine nach innen gerichtete Nocke 105 haben, die als ein der Spiralfeder 103 zugewandter Vorsprung ausgebildet ist. Die Nocke 105 wirkt mit einem nach außen vorstehend gebogenen Abschnitt 106 der Spiralfeder 103 zusammen, so größere Drehmomente übertragen werden können. Wenn die äußere Welle 103 blockiert, kann sich der gebogene Abschnitt 106 verformen und an der Nocke 105 vorbeirutschen.

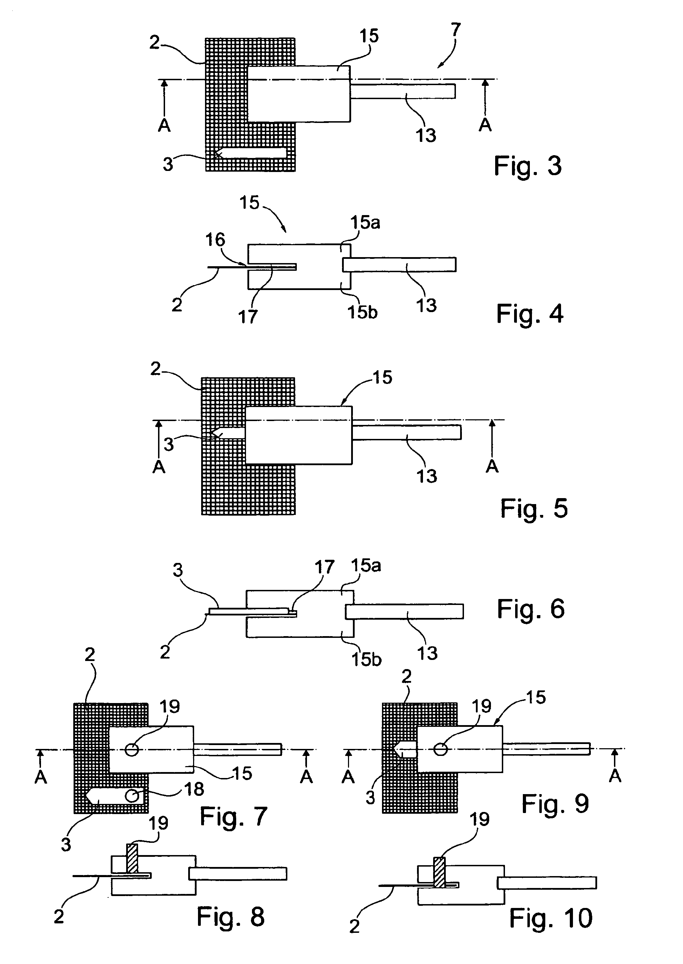

Eine Sperre, die einen Weitertransport des Trägerbandes 2 blockiert, sobald eine Lanzette 3 die Gebrauchsposition erreicht hat, kann auf unterschiedliche Weise verwirklicht werden. Ein erstes Ausführungsbeispiel für eine geeignete Sperre wird im Folgenden anhand der Figuren 3 bis 6 erläutert.

Figur 3 zeigt eine Detailansicht zu Figur 1, in der ein Abschnitt des Trägerbandes 2 mit einer Lanzette 3 vor Erreichen der Gebrauchsposition sowie einen Kopplungskopf 15 des Stechantriebs 7, mit dem eine von dem Stechantrieb 7 erzeugte Kraft für eine Stechbewegung auf eine Lanzette 3 und das mit ihr verbundene Trägerband 2 übertragen wird. Figur 4 zeigt eine Schnittdarstellung zu Figur 3 entlang der Schnittlinie A-A. Wie Figur 3 zeigt, hat der Kopplungskopf 15 einen Spalt 16, durch den das Trägerband 2 hindurchgeführt wird. Der Spalt 16 wird zwischen zwei gegeneinander beweglichen Teilen 15a, 15b des Kopplungskopfs 15 gebildet. Diese beiden Teile werden durch Federkraft gegeneinander gedrückt. Fertigungstoleranzen können so vorteilhaft ausgeglichen werden.

In der in Figur 3 dargestellten Lage der beiden Kopplungsteile 15a und 15b zueinander hat der Spalt 16 eine Engstelle, durch die das Trägerband 2, nicht jedoch eine an dem Trägerband 2 befestigte Lanzette 3 hindurchpasst. Sobald eine Lanzette 3 die Gebrauchsposition erreicht, wird sie und damit auch das mit ihr verbundene Trägerband 2 durch die Engstelle des Kopplungskopfs 15 blockiert. Dies ist in den Figuren 5 und 6 gezeigt.

Die Engstelle des Spaltes 16 wird durch eine Schulter mit einer Anschlagfläche 17 des Kopplungsteils 15a gebildet. In der Gebrauchsposition schlägt die Lanzette 3 an die Anschlagfläche 17 an. Das Kopplungsteil 15a mit der Anschlagfläche 17 bildet auf diese Weise ein Sperrelement, das mit einer Lanzette 3 in der Gebrauchsposition zusammenwirkt. Die Lanzette 3 hat einen Lanzettenkörper, der auf dem Trägerband 2 eine quer zur Förderrichtung verlaufende Stufe bildet, die als Blockadeelement zum Positionieren einer Lanzette 3 in der Gebrauchsposition mit der Anschlagfläche 17 der Sperre zusammenwirkt.

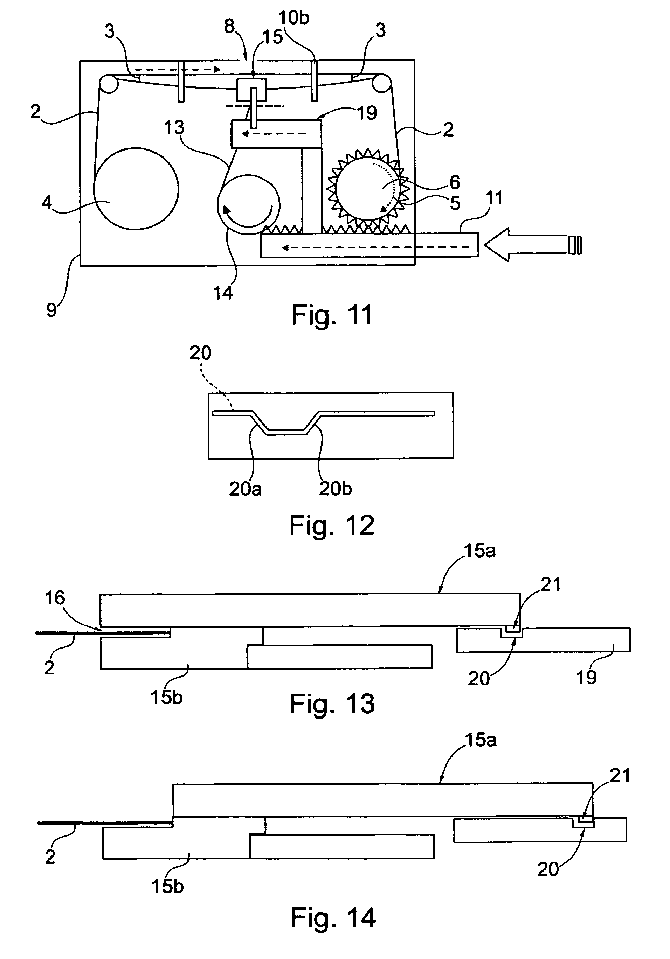

Beim Auslösen eines Stichs wird das Betätigungselement 11 in seine ursprüngliche Position zurückversetzt, beispielsweise mittels einer Rückstellfeder. Bei einer erneuten Betätigung des Betätigungselements 11 wird die Sperre gelöst, indem die beiden Kopplungselemente 15a und 15b gegeneinander bewegt werden, so dass sich der Spalt 16 vorübergehend verbreitert und das Trägeband 2 weiter transportiert werden kann. Ein Beispiel wie dies bewirkt werden kann, wird später anhand der Figuren 11 bis 14 erläutert.

Die Figuren 7 bis 10 zeigen ein weiteres Beispiel, wie eine Sperre bewirkt werden kann, die einen Weitertransport des Trägerbandes 2 blockiert, sobald eine Lanzette 3 die Gebrauchsposition erreicht hat. Bei diesem Beispiel ist in der Lanzette 3 ein Loch 18 als Blockadeelement vorgesehen, das mit einem als Zapfen ausgebildeten Sperrelement 19 zusammenwirkt, das in dem Kopplungsteil 15 angeordnet ist. Der Bolzen 19 ist senkrecht zur Ebene des Trägerbands 2 beweglich. Gelangt eine Lanzette 3 in die Gebrauchsposition, greift der Bolzen 19 in das Loch 18 der Lanzette 3 ein, so dass die Lanzette 3 und das mit ihr verbundene Trägerband 2 blockiert sind.

Um zuverlässig in das Loch 18 der Lanzette 3 einzugreifen kann der Bolzen 19 beispielsweise mit Federkraft gegen das Band 2 gedrückt sein. Eine Schrägfläche an seinem in das Loch 18 eingreifenden Ende bewirkt, dass der Bolzen 19 von einer in die Gebrauchsposition gelangenden Lanzette 3 angehoben werden kann. Sobald sich die Lanzette 3 in der Gebrauchsposition befindet, greift der Bolzen 19 dann in das Loch 18 der Lanzette 3 ein. Das Loch 18 der Lanzette 3 kann als Sackloch ausgebildet sein. Bevorzugt ist jedoch ein durchgehendes Loch.

Bei dem anhand der Figuren 7 bis 10 erläuterten Sperrmechanismus bildet der Bolzen 19 das Sperrelement der Sperre, das mit einer Lanzette 3 in der Gebrauchsposition formschlüssig zusammenwirkt. Die Umfangsfläche des Bolzens 19 bildet dabei eine Anschlagfläche, an die eine Lanzette 3 in der Gebrauchsposition anschlägt.

Indem ein Sperrelement der Sperre in den Kopplungskopf 15, der eine Bewegung des Stechantriebs auf das Trägerband 2 überträgt, integriert wird, kann eine besonders präzise Positionierung einer Lanzette 3 in der Gebrauchsposition erreicht werden, da dann nur bei wenigen Teilen Fertigungstoleranzen die Positioniergenauigkeit beeinflussen. Bei einem Sperrelement in dem Kopplungskopf 15 ist die Toleranzkette vorteilhaft kurz.

Das Loch 18 kann auch in dem Trägerband 2 neben einer Lanzette 3 angeordnet sein. Bei der Herstellung eines Trägerbandes 2 ist dann darauf zu achten, dass die Lanzetten 3 stets an einer vorgegebenen Position in Relation zu einem Loch 18 angeordnet sind. Dies kann dadurch erreicht werden, dass derartige Löcher erst nach dem Aufbringen der Lanzetten 3 in einer vorgegebenen Position in Relation zu den Lanzetten 3 in dem Trägerband 2 erzeugt werden. Möglich ist es auch, in einem Trägerband 2 zuerst Löcher zu erzeugen und die Lanzetten 3 in einer vorgegebenen Position relativ zu den Löchern anzuordnen.

Figur 11 zeigt eine weitere schematische Darstellung eines Stechsystems. Figur 11 unterscheidet sich von Figur 1 im wesentlichen durch einen Aktuator 19, der an die Betätigungseinrichtung 11 gekoppelt ist und dazu dient, die Sperre aus einer Sperrposition, in der ein Weitertransport des Förderbandes 2 blockiert wird, in eine Durchlassposition, in der Förderband 2 weitertransportiert werden kann, und wieder zurück in die Sperrposition zu überführen. Sperrpositionen sind beispielsweise in den Figuren 6 und 10 für unterschiedliche Ausführungsbeispiele der Sperre dargestellt.

Der Aktuator 19 ist bei dem dargestellten Ausführungsbeispiel über eine Kulissensteuerung an die Betätigungseinrichtung 11 gekoppelt. Ein Beispiel der Kulisse, die bei Betätigung der Betätigungseinrichtung 11 abgefahren wird, ist in Figur 12 gezeigt. Die Kulisse 20 bildet eine Steuerkurve, die von einem Steuerkurvenreiter abgefahren wird und dabei ein kurzes Anheben des als Bolzen 19 ausgebildeten Sperrelements bzw. eine Relativbewegung der beiden Kopplungsteile 15a, 15b zueinander bewirkt. Der Aktuator 19 wird gleichzeitig mit der Fördereinrichtung 6 von der Betätigungseinrichtung 11 betätigt, so dass während des kurzzeitigen Öffnens der Sperre eine benutzte Lanzette 3 aus der Gebrauchsposition weiterbewegt wird. Bevor eine frische Lanzette 3 die Gebrauchsposition erreicht, ist die Sperre in die Sperrposition zurückgekehrt, so dass die nächste Lanzette 3, sobald diese die Gebrauchsposition erreicht hat, zusammen mit dem mit ihr verbundenen Förderband 2 blockiert wird.

Figur 13 zeigt eine Schnittansicht des aus den beiden Teilen 15a, 15b gebildeten Kopplungskopfs 15 sowie des Aktuators 19 mit der als Nut ausgebildeten Kulisse 20. In die Kulisse 20 greift ein Stift 21 als Steuerkurvenreiter ein, der mit dem Kopplungsteil 15a verbunden ist. In Figur 13 ist die Sperre in der Sperrposition dargestellt. Beim Abfahren des Steuerkurvenabschnitts 20a der Kulisse 20 durch den Stift 21 vollzieht das Kopplungsteil 15a die durch die Kulisse 20 vorgegebene Bewegung nach und wird deshalb entgegen der Stichrichtung zurückgezogen, so dass die in Figur 14 gezeigte Position erreicht wird, in der das Trägerband 2 und eine von ihm getragene Lanzette 3 nicht mehr in dem Spalt 16 eingeklemmt sind. Durch den darauf folgenden Steuerkurvenabschnitt 20b wird die Sperre wieder in ursprüngliche Position, die in Figur 13 gezeigt ist, zurück versetzt.

Neben einer Kopplung des Aktuators an die Betätigungseinrichtung über eine zweiseitig geführte Kurvensteuerung, bei der ein Steuerkurvenreiter in eine Nut eingreift, besteht auch die Möglichkeit einer einseitig geführten Kurvensteuerung zur Kopplung des Aktuators an die Betätigungseinrichtung. Bei einer einseitig geführten Kurvensteuerung wird der Steuerkurvenreiter an eine Steuerkurve angedrückt, die beispielsweise eine Oberfläche mit Erhöhungen und Vertiefungen ist, über die der Steuerkurvenreiter beim Abfahren der Steuerkurve gleitet. Im Einfachsten Fall kann die Steuerkurve beispielsweise eine Rampe sein.

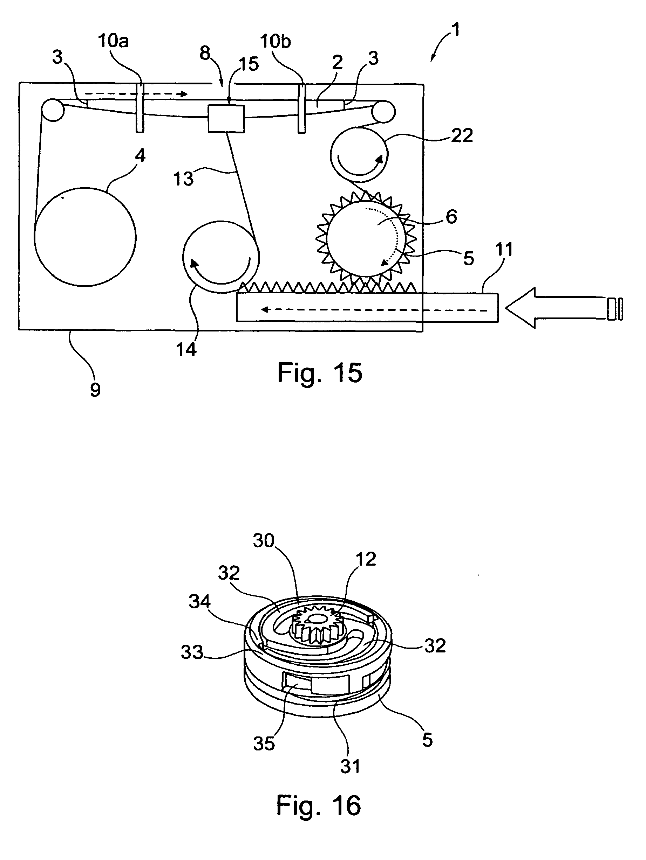

Figur 15 zeigt ein weiteres Ausführungsbeispiel eines Stechsystems 1. Dieses Ausführungsbeispiel unterscheidet sich von dem vorstehend erläuterten Ausführungsbeispiel dadurch, dass das Trägerband 2 mit einem Zählrad 22 gekoppelt ist, das sich bei einer durch die Fördereinrichtung 6 bewirkten Bewegung des Trägerbandes 2 zur Messung einer von dem Trägerband 2 zurückgelegten Wegstrecke dreht und blockiert, sobald die gemessene Wegstrecke die zum Positionieren einer Lanzette 3 in der Gebrauchsposition erforderliche Länge erreicht hat. Das Zählrad 22 bildet bei diesem Ausführungsbeispiel also die Sperre. Bevorzugt ist der Umfang des Zählrads 22 so bemessen, dass er genau den Abstand zwischen benachbarten Lanzetten 3 auf dem Trägerband 2 entspricht und das Zählrad 22 deshalb jeweils nach einer vollen Umdrehung sperrt. Möglich ist es selbstverständlich auch, dass das Zählrad 22 bei einer beliebigen anderen vorgegebenen Drehwinkelstellung oder einer vorgegebenen Anzahl von Umdrehungen sperrt. Durch Reibschluss oder eine mit dem Zählrad 22 zusammenwirkende Perforation des Trägerbandes 2 kann Schlupf des Trägerbandes 2 vermieden werden.

Figur 16 zeigt ein Ausführungsbeispiel eines Freilaufmoduls 30 das bei dem in Figur 1 dargestellten Ausführungsbeispiel anstelle einer Rutschkupplung 100 eingesetzt werden kann. Das Freilaufmodul 30 hat ein Zahnrad 12, über das zum Spannen einer Torsionsfeder 31 eine Drehbewegung erzeugt werden kann, beispielsweise mittels einer in Figur 1 dargestellten Zahnstange 11. Bei dem dargestellten Ausführungsbeispiel koppelt das Zahnrad 12 bei einer Drehung in einer ersten Drehrichtung (in Figur 16 im Uhrzeigersinn) mittels elastischer Zungen 32 an einen umgebenden Ring 33, indem die Zungen 32 an Stufen 34 anstoßen. Der Ring 33 ist mit der Torsionsfeder 31 gekoppelt, so dass durch Drehen des Rings 33 in der ersten Drehrichtung die Torsionsfeder 31 gespannt wird, die auf die Wickelrolle 5 einwirkt.

Bei Erreichen eines fest vorgegebenen Drehwinkels wird der Ring 33 arretiert, beispielsweise formschlüssig. Bei dem in Figur 16 gezeigten Ausführungsbeispiel sind in einer Außenfläche des Rings 33 Ausnehmungen 35 vorgesehen, in die Rastelemente eines Ratschenmechanismus zum Arretieren des Rings 33 eingreifen können.

Die Torsionsfeder 31, die in der Regel als Spiralfeder ausgebildet ist, kann sich nun nur noch durch eine Drehung der Wickelrolle 5 entspannen, was jedoch nur möglich ist, wenn die an Figur 1 erläuterte lösbare Sperre geöffnet ist, d.h. keine Lanzette 3 an dem Spalt 16 des Kopplungskopfes 15 anschlägt. Sobald die lösbare Sperre geöffnet wird, entspannt sich die Torsionsfeder 31 zumindest teilweise und bewirkt eine Drehbewegung der Wickelrolle 5 bis eine frische Lanzette 3 in die Gebrauchsposition gelangt ist und die Sperre erneut blockiert.

Wird die Zahnstange 11 nach dem Spannen der Torsionsfeder in ihre ursprüngliche Lage zurückgeführt, dreht sich das Zahnrad 12 in einer zweiten Drehrichtung, bei dem gezeigten Ausführungsbeispiel gegen den Uhrzeigersinn. Dabei gleiten die Zungen 32 über die Stufen 34 des Rings 33 hinweg, so dass sich dieser nicht mitdreht. Eine Rückführfeder kann vorgesehen sein, um die Zahnspange 11 selbststätig in ihre Ausgangsposition zurückzubewegen.

Wie bereits erläutert, hängt der Winkel, um den sich die Wickelrolle 5 drehen muss, um eine frische Lanzette in die Gebrauchsposition zu bringen, davon ab, wie viel Trägerband bereits auf die Wickelrolle 5 gewickelt wurde, da dies den effektiven Durchmesser der Wicklung beeinflusst. Die mit einem Spannvorgang bewirkte Torsion der Torsionsfeder stimmt deshalb allenfalls zufällig mit der für einen Transportschritt benötigen Torsion überein.

Diesem Problem kann beispielsweise dadurch begegnet werden, dass die Zahnstange 11 mehrmals gedrückt werden muss, um eine Lanzette in die Gebrauchsposition zu bringen, beispielsweise zweimal bei einem frischen Band und folglich kleinem Durchmesser der Wickelung und nur einmal gegen Ende eines Bandes und folglich großem Durchmesser der Wickelung. Die Kopplung eines solchen Bedienschrittes mit dem Spannen des Stechantriebs ist jedoch schwierig, da die entsprechenden Hubbewegungen nicht übereinstimmen und schwer zu synchronisieren sind. Ausweg bietet eine Ausführungsform, bei der die Feder des Transportantriebs derart vorgespannt ist, dass diese Vorspannung in der Mittellage (Vorratsrolle 4 und Wickelrolle 5 haben dann den gleichen Durchmesser) aufgezehrt ist. Bei kleinem Durchmesser der Wickelrolle 5 wird mehr Drehwinkel für den Bandtransport benötigt und entsprechend zusätzliche Energie für den Vortrieb aus der Vorspannung entnommen. Bei größerem Durchmesser der Wickelrolle kehrt sich die Situation um: jeder Spannvorgang erzielt nun einen überschüssigen Energiebetrag, der von der Torsionsfeder aufsummiert wird. Im Idealfall erreicht dieser aufsummierte Beitrag mit der letzten Lanzette die Höhe der initialen Federvorspannung. Diese Vorspannung kann werkseitig realisiert werden, beispielsweise bei Einmalgeräten, oder beim Einlegen einer Bandkassette von einem Beladesystem erzeugt werden. Vorteilhaft ist dabei, dass der Drehwinkel beim Spannen der Torsionsfeder 31 fest vorgegeben werden kann. Somit lässt sich ein konstanter Hub der Zahnstange realisieren, der sich einfach mit dem Spannen des Stechantriebs synchronisieren lässt. Hierdurch wird ein einfach zu bedienendes Gerät mit extrem wenig Bedienelementen ermöglicht.

Eine andere Möglichkeit besteht darin, die Torsionsfeder so auszulegen, dass diese sich nur bei Einlegen eines neuen Trägerbandes vollständig entspannt und die Federspannung mit den einzelnen Transportschritten etwas zunimmt, da im Regelfall die mit einem Spannvorgang bewirkte Torsion nur teilweise bei einem anschließenden Transportschritt verbraucht wird.

### Bezugszahlenliste

- 1: Stechsystem
- 2: Trägerband
- 3: Lanzette
- 4: Vorratsrolle
- 5: Wickelrolle
- 6: Fördereinrichtung
- 7: Stechantrieb
- 8: Gehäuseöffnung
- 9: Gehäuse
- 10a: Umlenkeinrichtung
- 10b: Umlenkeinrichtung
- 11: Betätigungseinrichtung
- 12: Zahnrad
- 13: Pleuelstange
- 14: Rad
- 15: Kopplungskopf
- 15a: Teil des Kopplungskopfs
- 15b: Teil des Kopplungskopfs
- 16: Spalt
- 17: Anschlagfläche
- 18: Loch
- 19: Aktuator
- 20: Kulisse
- 20a: Steuerkurvenabschnitt
- 20b: Steuerkurvenabschnitt
- 21: Stift
- 22: Zählrad
- 30: Freilaufmodul
- 31: Torsionsfeder
- 32: Zungen
- 33: Ring
- 34: Stufen
- 35: Ausnehmungen
- 100: Rutschkupplung
- 101: innere Welle
- 102: äußere Welle
- 103: Spiralfeder
- 104: inneres Ende der Spiralfeder
- 105: Nocken
- 106: vorstehend gebogener Abschnitt der Spiralfeder

## Patentansprüche

1. Stechsystem mit
einem Trägerband (2), das mehrere Lanzetten (3) trägt,
einem Gerätegehäuse (9), das eine Fördereinrichtung (6), um die Lanzetten (3) durch Bewegen des Trägerbandes (2) in einer Förderrichtung nacheinander in eine Gebrauchsposition zu bringen, und einen Stechantrieb (7), um eine in der Gebrauchsposition positionierte Lanzette (3) für eine Stechbewegung zu beschleunigen, enthält und eine Gehäuseöffnung (8) zum Anlegen eines Körperteils, in das mit einer Stechbewegung einer Lanzette (3) eingestochen werden soll, aufweist,
eine Betätigungseinrichtung (11) zum Antreiben der Fördereinrichtung (6), und
eine lösbare Sperre (15,17),
**dadurch gekennzeichnet, dass**
die Sperre (15,17) einen Anschlag (17 ) aufweist, der ausgelegt ist, einen Weitertransport des Trägerbandes (2) zu blockieren sobald eine Lanzette (3) die Gebrauchsposition erreicht hat.

2. Stechsystem nach Anspruch 1, **dadurch gekennzeichnet, dass** die Sperre (17, 18, 21) durch erneutes Betätigen der Betätigungseinrichtung (11) lösbar ist.

3. Stechsystem nach einem der vorstehenden Ansprüche, **gekennzeichnet durch** eine Rutschkupplung (100), über welche die Betätigungseinrichtung (11) an die Fördereinrichtung (6) gekoppelt ist,

4. Stechsystem nach einem der vorstehenden Ansprüche, **dadurch gekennzeichnet, dass** der Antrieb einen Energiespeicher umfasst, der die für eine Stechbewegung erforderliche Energie liefert und mit der Betätigungseinrichtung (11) gekoppelt ist, so dass durch Betätigen der Betätigungseinrichtung (11) ein Aufladen des Energiespeichers bewirkt wird.

5. Stechsystem nach einem der vorstehenden Ansprüche, **dadurch gekennzeichnet, dass** die Sperre ein Sperrelement (17, 18) umfasst, das mit einer Lanzette (3) in der Gebrauchsposition zusammenwirkt.

6. Stechsystem nach Anspruch 5, **dadurch gekennzeichnet, dass** das Sperrelement (17, 18) den mit einer Lanzette (3) in der Gebrauchsposition formschlüssig zusammenwirkt.

7. Stechsystem nach Anspruch 5 oder 6, **dadurch gekennzeichnet, dass** das Sperrelement (17, 18) eine Anschlagfläche aufweist, an die eine Lanzette (3) in der Gebrauchsposition anschlägt.

8. Stechsystem nach einem der vorstehenden Ansprüche, **dadurch gekennzeichnet, dass** die Sperre (17, 18, 22) mittels eines an die Betätigungseinrichtung (11) gekoppelten Aktuators (19) aus einer Sperrposition, in der ein Weitertransport des Förderbandes (2) blockiert wird, in eine Durchlassposition, in der das Förderband (2) weitertransportiert werden kann, überführbar ist.

9. Stechsystem nach Anspruch 8, **dadurch gekennzeichnet, dass** der Aktuator (19) über eine Kurvensteuerung (20) an die Betätigungseinrichtung (11) gekoppelt ist.

10. Stechsystem nach einem der vorstehenden Ansprüche, **dadurch gekennzeichnet, dass** der Stechantrieb (7) einen Kopplungskopf (15) umfasst, der einen Spalt (16) aufweist, durch den das Trägerband (2) hindurchgeführt ist, wobei die lösbare Sperre (17) eine Engstelle des Spalts (16) bildet.

11. Stechsystem nach einem der vorstehenden Ansprüche, **dadurch gekennzeichnet, dass** die Sperre ein mit dem Trägerband (2) gekoppeltes Zählrad (22) umfasst, das sich bei einer durch die Fördereinrichtung (6) bewirkten Bewegung des Trägerbandes (2) zur Messung einer von dem Trägerband (2) zurückgelegten Wegstrecke dreht und blockiert, sobald die Wegstrecke die zum Positionieren einer Lanzette (3) in der Gebrauchsposition erforderliche Länge erreicht hat.

12. Stechsystem nach einem der vorstehenden Ansprüche, **dadurch gekennzeichnet, dass** die Betätigungseinrichtung (11) eine Stange umfasst, die zur Betätigung der Fördereinrichtung (6) von einem Benutzer in ihrer Längsrichtung verschoben wird.

13. Stechsystem nach einem der vorstehenden Ansprüche, **dadurch gekennzeichnet, dass** die Fördereinrichtung (6) eine Antriebsfeder umfasst.

14. Stechsystem nach einem der vorstehenden Ansprüche, **dadurch gekennzeichnet, dass** die Betätigungseinrichtung (11) über ein Freilaufmodul (30) an die Fördereinrichtung (6) gekoppelt ist.

## Claims

1. Puncturing system comprising
a carrier tape (2) that carries multiple lancets (3),
a device housing (9) that contains a conveying facility (6) for moving the lancets (3) into a usage position consecutively by moving the carrier tape (2) in a conveying direction, and a puncturing drive (7) for accelerating a lancet (3) that is positioned in the usage position for a puncturing motion, and has a housing opening (8) for touching against a body part, in which a puncture is to be made by a puncturing motion of a lancet (3),
an actuation facility (11) for driving the conveying facility (6), and
a releasable detent,
**characterized in that**
the detent comprises a stop element (17) that is configured to block further transport of the carrier tape (2) as soon as a lancet (3) has reached the usage position.

2. Puncturing system according to claim 1, **characterized in that** the detent (17, 18, 21) can be unlocked by again actuating the actuation facility (11).

3. Puncturing system according to any one of the preceding claims, **characterized by** a slipping clutch (100) via which the actuation facility (11) is coupled to the conveying facility (6).

4. Puncturing system according to any one of the preceding claims, **characterized in that** the drive includes an energy storage that supplies the energy required for a puncturing motion and is coupled to the actuation facility (11) such that the energy storage is charged by actuating the actuation facility (11).

5. Puncturing system according to any one of the preceding claims, **characterized in that** the detent comprises an interlocking element (17, 18) that acts in conjunction with a lancet (3) in the usage position.

6. Puncturing system according to claim 5, **characterized in that** the interlocking element (17, 18) acts in conjunction with a lancet (3) in the usage position in a form-fitting manner.

7. Puncturing system according to claim 5 or 6, **characterized in that** the interlocking element (17, 18) has a stop surface against which a lancet (3) abuts in the usage position.

8. Puncturing system according to any one of the preceding claims, **characterized in that** the detent (17, 18, 22) can be switched from an interlocking position, in which further transport of the conveyor tape (2) is blocked, to a pass-through position, in which the conveyor tape (2) can be transported further, by an actuator (19) that is coupled to the actuation facility (11).

9. Puncturing system according to claim 8, **characterized in that** the actuator (19) is coupled to the actuation facility (11) by a cam control (20).

10. Puncturing system according to any one of the preceding claims, **characterized in that** the puncturing drive (7) comprises a coupling head (15) that has a gap (16) through which the carrier tape (2) is guided, whereby the releasable detent (17) forms a constriction of the gap (16).

11. Puncturing system according to any one of the preceding claims, **characterized in that** the detent comprises a counting wheel (22) that is coupled to the carrier tape (2) and rotates upon a motion of the carrier tape (2) that is effected by the conveying facility (6), to measure a distance traveled by the carrier tape (2), and is blocked as soon as the distance has reached the length required to position a lancet (3) in the usage position.

12. Puncturing system according to any one of the preceding claims, **characterized in that** the actuation facility (11) comprises a rack that is shifted in its longitudinal direction by a user in order to actuate the conveying facility (11).

13. Puncturing system according to any one of the preceding claims, **characterized in that** the conveying facility (6) comprises a drive spring.

14. Puncturing system according to any one of the preceding claims, **characterized in that** the actuation facility (11) is coupled to the conveying facility (6) by a free-wheel module (30).

## Revendications

1. Système de piqûre comportant
une bande de support (2), qui supporte plusieurs lancettes (3),
un boîtier d'appareil (9), qui renferme un dispositif de transport (6) afin de mettre les lancettes (3) les unes derrière les autres dans une position d'utilisation dans une direction de transport par un mouvement de la bande de support (2), et un dispositif d'entraînement de piqûre (7) afin d'accélérer une lancette (3) positionnée dans la position d'utilisation pour un mouvement de piqûre et présente une ouverture de boîtier (8) afin de positionner une partie de corps, dans laquelle une piqûre doit être effectuée avec un mouvement de piqûre d'une lancette (3),
un dispositif d'actionnement (11) afin d'entraîner le dispositif de transport (6), et un dispositif de blocage détachable (15, 17),
**caractérisé en ce que**
le dispositif de blocage (15, 17) présente une butée (17), qui est prévue pour bloquer un transport supplémentaire de la bande de support (2) dès qu'une lancette (3) a atteint la position d'utilisation.

2. Système de piqûre selon la revendication 1, **caractérisé en ce que** le dispositif de blocage (17, 18, 21) est détachable par le biais d'un actionnement renouvelé du dispositif d'actionnement (11).

3. Système de piqûre selon l'une des revendications précédentes, **caractérisé par** un accouplement à friction (100), par le biais duquel le dispositif d'actionnement (11) est accouplé au dispositif de transport (6).

4. Système de piqûre selon l'une des revendications précédentes, **caractérisé en ce que** le dispositif d'entraînement comprend un réservoir d'énergie qui délivre l'énergie nécessaire pour un mouvement de piqûre et qui est accouplé au dispositif d'actionnement (11), de sorte que par le biais de l'actionnement du dispositif d'actionnement (11) un rechargement du réservoir d'énergie est effectué.

5. Système de piqûre selon l'une des revendications précédentes, **caractérisé en ce que** le dispositif de blocage comprend un élément de blocage (17, 18) qui coopère avec une lancette (3) dans la position d'utilisation.

6. Système de piqûre selon la revendication 5, **caractérisé en ce que** l'élément de blocage (17, 18) coopère par complémentarité de formes avec une lancette (3) dans la position d'utilisation.

7. Système de piqûre selon la revendication 5 ou 6, **caractérisé en ce que** l'élément de blocage (17, 18) présente une surface de butée contre laquelle bute une lancette (3) dans la position d'utilisation.

8. Système de piqûre selon l'une des revendications précédentes, **caractérisé en ce que** le dispositif de blocage (17, 18, 22) peut passer au moyen d'un actionneur (19) accouplé au dispositif d'actionnement (11) d'une position de blocage, dans laquelle un transport supplémentaire de la bande de transport (2) est bloqué, à une position de passage, dans laquelle la bande de transport (2) peut continuer à être transportée.

9. Système de piqûre selon la revendication 8, **caractérisé en ce que** l'actionneur (19) est accouplé par le biais d'une commande de courbe (20) au dispositif d'actionnement (11).

10. Système de piqûre selon l'une des revendications précédentes, **caractérisé en ce que** le dispositif d'entraînement de piqûre (7) comprend une tête d'accouplement (15) qui présente un interstice (16) à travers lequel passe la bande de support (2), le dispositif de blocage détachable (17) formant un rétrécissement de l'interstice (16).

11. Système de piqûre selon l'une des revendications précédentes, **caractérisé en ce que** le dispositif de blocage comprend une roue dentée (22) accouplée à la bande de transport (2), roue qui se tourne et se bloque lors d'un mouvement de la bande de transport (2) provoqué par le dispositif de transport (6) afin de mesurer une distance parcourue par la bande de transport (2), dès que la distance a atteint la longueur nécessaire au positionnement d'une lancette (3) dans la position d'utilisation.

12. Système de piqûre selon l'une des revendications précédentes, **caractérisé en ce que** le dispositif d'actionnement (11) comprend une barre qui est déplacée dans sa direction longitudinale par un utilisateur afin d'actionner le dispositif de transport (6).

13. Système de piqûre selon l'une des revendications précédentes, **caractérisé en ce que** le dispositif de transport (6) comprend un ressort d'entraînement.

14. Système de piqûre selon l'une des revendications précédentes, **caractérisé en ce que** le dispositif d'actionnement (11) est accouplé au dispositif de transport (6) par le biais d'un module de roue libre (30).
